# EUROPEAN PATENT SPECIFICATION

(11) **EP 1 726 591 B1**
(45) Date of publication and mention of the grant of the patent: **10.09.2008**
(21) Application number: 06010476.7
(22) Date of filing: 22.05.2006
(51) Int. Cl.: C07D 405/12

(54) **Process for manufacturing paroxetine hydrochloride hemihydrate**
Verfahren zur Herstellung von Paroxetin Hydrochlorid Hemihydrat
Procedé de la Fabrication de l'Hemihydrate de l'Hydrochlorure de Paroxetine

(30) Priority: 26.05.2005 US 685237 P
(43) Date of publication of application: 29.11.2006
(73) Proprietor: Apotecnia , S.A., 33192 Llanera (ES)
(72) Inventor: Segnalini, Franca, 104-04100 Latina (IT); Magnanti, Stefano, 00185 Rome (IT)
(74) Representative: Isern-Jara, Nuria

(56) References cited:
- WO-A-00/32593
- WO-A-01/14335
- WO-A-20/07054978
- US-A- 4 721 723
- US-A- 5 872 132
- US-A1- 2003 158 230
- BUXTON P C ET AL: "SOLID-STATE FORMS OF PAROXETINE HYDROCHLORIDE" 1988, INTERNATIONAL JOURNAL OF PHARMACEUTICS (KIDLINGTON), VOL. 42, NR. 1-3, PAGE(S) 135-144 , XP002399861 ISSN: 0378-5173 * the whole document *

## Description

### Field of the invention

The present invention relates to a new process for manufacturing paroxetine hydrochloride hemihydrate and, more particularly, to a process which comprises converting a crystalline anhydrous solvate of paroxetine hydrochloride into the corresponding crystalline hemihydrate in the solid state. This process is substantially different from the dissolution - precipitation sequence described in the known art.

### State of the art

Paroxetine (Merck Index, 1996, n. 7175) is a well known antidepressant drug described for the first time in the US patent US4007196 (Ferrosan), as an amorphous free base (col. 6, lines 67-68) and as a crystalline maleate (col. 7, lines 2-5), of formula

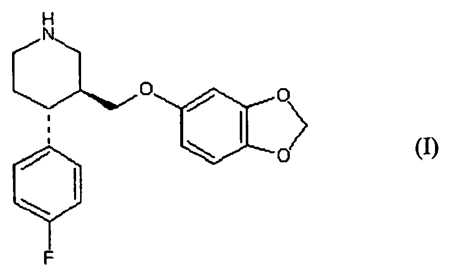

Several other pharmaceutical acceptable salts of paroxetine such as, for example, the acetate, mesylate, hydrochloride, and in particular, the hydrochloride hemihydrate, are known in the literature.

The crystalline hydrochloride hemihydrate is preferably used instead of amorphous or crystalline anhydrous hydrochlorides, in the marketed oral formulations of paroxetine (Paxil® Seroxat®, because of its better properties, namely its superior stability and much lower hygroscopicity.

The patent US4721723, in the name of Beecham, describes some processes for manufacturing paroxetine hydrochloride hemihydrate, processes which are substantially characterized by providing a solution of paroxetine hydrochloride in organic solvents and, subsequently, by crystallizing the hydrochloride hemihydrate from said solution. This patent discloses a number of process variants, in which the solution of paroxetine hydrochloride is prepared:
- by dissolving a pre-formed paroxetine hydrochloride, or
- by preparing the hydrochloride in situ, namely by treating a solution of paroxetine free base or of a salt thereof, different from the hydrochloride, with gaseous hydrochloric acid or its aqueous and/or organic solutions. The crystallization of the hydrochloride hemihydrate is effected with or without seeding, depending on, e.g. the purity of the starting paroxetine or the solvent selected for the crystallization. Obviously, in order to obtain the hemihydrate, some water must be present in the system, at least in a stoichiometric amount. Such water may be contained in the crystallization solvent ― because said solvent is not anhydrous or water/organic solvent admixtures are used - or may be added during the salification step as a concentrated or diluted aqueous solution of hydrochloric acid.

Nevertheless the unavoidable presence of water in the crystallization solvent causes some significant drawbacks.

Firstly there is co-precipitation of lipophilic impurities and byproducts, such as for example N-methyl, N-ethyl or N-benzyl paroxetine, said byproducts being intermediates commonly used during paroxetine synthesis, with contamination of the final paroxetine hydrochloride hemihydrate.

In order to achieve the high purity standards of Pharmacopeias, it becomes thus mandatory to further purify the product, for example by subsequent crystallizations in more lipophilic solvents or according to other common techniques, invariably reducing yields and increasing time and costs of the overall process.

Another disadvantage associated with the presence of water into the crystallization system, especially if said water is contained in a significant amount, is a reduction in the crystallization yields of paroxetine hydrochloride hemihydrate due to a partial solubility of this product in water.

Other processes for manufacturing crystalline paroxetine hydrochloride hemihydrate are known from the literature, processes which differ from the classical crystallization procedure described above in that they do not provide for dissolution and precipitation of the desired product but, on the contrary, they effect the conversion within polymorphs or pseudo polymorphs in the solid state.

For example, the above discussed patent, in the name of Beecham, shows an alternative process ― scarcely applicable on an industrial scale ― for manufacturing crystalline paroxetine hydrochloride hemihydrate by compression of anhydrous crystalline paroxetine hydrochloride (col. 3, lines 19-22 and col. 10, lines 14-18).

Another method for preparing crystalline paroxetine hydrochloride hemihydrate, suggested in the same Beecham's patent (see col. 10, lines 19-22) and better described in International Journal of Pharmaceuticals, 42, (1988), 135-143, provides for the conversion of anhydrous crystalline paroxetine hydrochloride solvate with 2-propanol, named Form II, in crystalline paroxetine hydrochloride hemihydrate, named Form I, by exposing said Form II to a moist atmosphere, in the presence of crystallization seeds. The conversion is said to be completed if Form II sample is seeded with 1 to 5% of Form I and maintained at 37°C/ 75% RH for 7 days (page 139, paragraph: *Interconversion of* ...).

However also this process is not industrially applicable because very low and not reproducible: in scaling up this step the time required for the conversion would be too long to be of any practical interest.

The patent US5872132, also in the name of Beecham, does not relate to the preparation of crystalline paroxetine hydrochloride hemihydrate but, on the contrary, describes the manufacturing of crystalline anhydrous paroxetine hydrochloride substantially devoid of bound solvent, manufacturing which includes the step of removing said bound solvent ― meaning the solvent bound within the crystal lattice and not removable by simple drying ― from a crystalline anhydrous solvate of paroxetine hydrochloride. According to the description, the bound solvent can be removed from the solvate by displacement with a suitable agent, such as water or supercritical carbon dioxide or other common agents. When the displacing agent is water, gaseous or liquid, it is stated that: "... *paroxetine hydrochloride solvate is contacted with enough water and for a sufficient time to displace the solvent but insufficient to cause conversion to the hydrochloride hemihydrate* " (col. 5, lines 26-29).

However in this patent no confirmation or practical suggestion either on the feasibility of the conversion to the hydrochloride hemihydrate by treatment with water or on the industrial applicability of such a process are given.

On the contrary the literature provides for some discouraging indications which would put off the skilled in the art, especially in view of a final industrial application.

For example US5872132 states that after 10 minutes (ex. 6, acetonitrile solvate; ex. 9, acetone solvate; ex. 10, ethanol solvate; ex. 11, chloroform solvate; ex. 13, 1-propanol solvate) or, indeed, 20 minutes (ex. 1, 2-propanol solvate) of suspension in water under stirring of the anhydrous paroxetine hydrochloride solvate, there is only displacement ― and not substitution by water - of the bound solvent, thus providing the desolvated form which contains superficial water not steadily bound within the crystal lattice.

In Chem. Pharm. Bull. (2000), 48(4), 529-536 it is stated that crystalline anhydrous paroxetine hydrochloride solvate with 2-propanol (form II) left in the open air at room temperature for 72 hours (figures 3a and 3b, at page 531) adsorbs water slowly and then releases it easily under anhydrous atmosphere, meaning that this water is not bounded but only superficially adsorbed (pages 530-531).

We have now surprisingly found an industrially applicable process for manufacturing paroxetine hydrochloride hemihydrate by hydration of anhydrous solvates of paroxetine hydrochloride, in particular of 2-propanol solvate, in the solid state.

Additionally we have found that the manufacture of paroxetine hydrochloride hemihydrate if performed according to the process of the present invention - namely by dissolution of the base, precipitation of the anhydrous hydrochloride solvate under anhydrous conditions followed by its conversion into the hemihydrate in the solid state ― provides for a product with higher purity and yields if compared with the classical procedure ― namely by dissolution of the same base and precipitation of the hydrochloride hemihydrate under non-anhydrous conditions - with conversion times and experimental conditions well suitable for the industrial application.

### Summary of the invention

It is thus an object of the present invention a process for manufacturing crystalline paroxetine hydrochloride hemihydrate which comprises the steps of:
a) wetting a crystalline anhydrous solvate of paroxetine hydrochloride, said solvate preferably comprising a solvent selected form the group consisting of methanol, ethanol, 1-propanol, 2-propanol, 1-butanol, acetone, acetonitrile, acetic acid and pyridine, with water or admixtures thereof with water-miscible solvents, and
b) heating the wet crystalline solvate of paroxetine hydrochloride, at a temperature between 30 and 60°C, up to its complete conversion into crystalline paroxetine hydrochloride hemihydrate.

### Detailed description of the invention

The crystalline anhydrous solvate of paroxetine hydrochloride, starting material of the present process, preferably comprises a solvent selected from the group consisting of methanol, ethanol, 1-propanol, 2-propanol, 1-butanol, acetone, acetonitrile, acetic acid and pyridine, being the 2-propanol solvate particularly preferred.

These solvates, generally, can be prepared according to the experimental description of US5872132, herein incorporated by reference, starting from paroxetine base, prepared for example as described in US6583287.

In the specific case of 2-propanol solvate, it may be preferably prepared as described in CA2187128 (example 1).

In the present process the crystalline anhydrous solvate of paroxetine hydrochloride may be used as such or, preferably, it may undergo a partial drying, for example in the case of 2-propanol solvate, up to a 2-propanol content lower than 20% w/w, preferably lower than 15% w/w.

The crystalline anhydrous solvate is then wetted with water or admixtures thereof with water-miscible solvents, preferably with water.

When admixtures of water with water-miscible solvents are used, said solvent is preferably selected form the group consisting of methanol, ethanol, 1-propanol, 2-propanol, 1-butanol, acetone, acetonitrile, acetic acid and pyridine. The choice of the solvent will substantially depend on the nature of the solvate, being preferred the same solvent included within the crystal lattice of the anhydrous solvate. For example, in the case of the 2-propanol solvate, admixtures of 2-propanol and water will be used, being however always preferred the use of water alone.

The admixtures with water-miscible solvents will comprise at least about 5% w/w of water, preferably at least 10% w/w, more preferably at least 20% w/w.

With the term "wetting" it should be meant intimately contacting the crystalline anhydrous solvate with water, namely making water absorbed on the surface of the solid without dissolution of the same. Such operation may be performed, for example, by washing the solid solvate, placed into a filtering device, with water or admixtures thereof, or by rapid suspension of the same under stirring in water or admixtures thereof followed by filtration. Preferably the selected crystalline anhydrous solvate is wetted by washing on a filter. In such a case, the number of washings, their duration, the amount of the wetting admixture for each washing will be selected as to obtain, after washing, a soaked cake with a rather high content of water, preferably in the range of about 20 - 45% w/w.

The wetting solvents - namely water or its admixtures with water-miscible solvents- are usually at room temperature or, preferably, they are previously cooled at lower temperature, preferably at a temperature lower than 20°C, more preferably at about or lower than 10 °C.

The wet crystalline solvate of paroxetine hydrochloride is then heated, generally at room pressure, in the air or, preferably, under an inert atmosphere, for example under nitrogen, or under vacuum, under a temperature gradient or, preferably, at a constant temperature, said temperature being from 30°C to 60°C, preferably from 35 to 45°C, more preferably at about 40°C up to its conversion into crystalline paroxetine hydrochloride hemihydrate.

Preferably, the wet solvate is heated in a closed system, under a water saturated atmosphere, according to conventional techniques, for example by transferring the solid into an oven or in a static dryer or, alternatively, by direct heating into the filtration device, for example in heated filter-dryer of Comber or CMR type, or in equivalent systems up to completion of the conversion process.

According to the process of the present invention the obtained crystalline paroxetine hydrochloride hemihydrate may have a granulometry, expressed as D₉₀, generally from 10 to 20 µm, which is pharmaceutically interesting for its high dissolution rate and bioavailability. However, the skilled in the art will be able to obtain powders with a higher granulometry in order to satisfy other technological requirements, such as for example powder flowability, compression properties etc.., by suitably modifying the operating conditions of the filter-dryers during the conversion and/or drying steps, for example by varying temperatures, times or mixing speed.

The conversion of the anhydrous solvates into paroxetine hydrochloride hemihydrate, according to the preset invention and differently from what stated in International Journal of Pharmaceuticals, 42, (1988), 135-143, is effected even without seeding with crystals of paroxetine hydrochloride hemihydrate, and it is generally completed within industrially acceptable times. The duration of the heating will vary, depending on several factors such as the kind and the amount of the solvate, the composition of the wetting admixture and the amount of water retained in the cake, the mixing conditions, the temperature ...

At the end of the conversion, the product usually undergoes a drying step, by heating under vacuum, in order to remove the exceeding water from the surface and the residual traces of the displaced solvent. Preferably, the final drying is performed by progressive heating under stirring, thus minimizing the formation of lumps of product along the walls of the dryer.

It is then possible to check that said conversion occurred by DSC, IR, Karl Fischer analysis (theoretical water content equal to 2.4% w/w) in comparison with the standard data from Pharmacopeias.

Crystalline paroxetine hydrochloride hemihydrate, prepared according to the present invention, is stable when exposed to the air after drying: in fact samples of the dried product ― prepared by conversion of crystalline anhydrous paroxetine hydrochloride solvate with 2-propnol ― left in the open air for 11 days have maintained a K.F.= 2,4-2,7% and a 2-propanol content lower than 2000 ppm, thus confirming a strong binding of the water within the crystal lattice and an almost complete displacement of the solvent.

In a particularly preferred embodiment oily paroxetine base containing toluene, is dissolved in anhydrous 2-propanol under stirring, at room temperature. An excess of a solution of hydrochloric acid in anhydrous 2-propanol is then added dropwise and the admixture is seeded with crystals of paroxetine hydrochloride 2-propanol solvate. There is precipitation of the solvate which, after cooling, is filtered, washed with pre-cooled anhydrous 2-propanol and partially dried.

The solvate, humid with 2-propanol, is copiously washed on a filter-dryer with pre-cooled water. The wet product is heated at about 40°C, under nitrogen, for 3-5 hours, namely up to complete conversion, then dried thus providing paroxetine hydrochloride hemihydrate complying with Pharmacopeias reference standards.

With the aim of better illustrating the present invention, without limiting it, the following examples are now given.

### EXPERIMENTAL PART

Analysis: paroxetine hydrochloride hemihydrate has been analyzed according to the methods described in US Pharmacopeia, in particular: HPLC: column Symmetry RP8, Detector UV-Vis at 295 nm, Flux 1 ml/min, T 40°C, Elution gradient, (RT 24.4 min)

FT-IR (reflection), characteristic peaks (cm⁻¹):
3339.5, 2818.6, 2120.6, 1899.0, 1837.2, 1605.9, 1511.0, 1479.7, 1392.5, 1380.9, 1345.1, 1278.5, 1221.5, 1184.6, 1129.1, 1097.2, 1064.5, 1041.5, 1015.9, 930.6, 892.1, 836.2, 805.3, 780.6, 736.9, 715.0, 675.1, 598.0, 573.4, 541.4, 450.0, 427.0

### EXAMPLE 1

### Preparation of crystalline anhydrous paroxetine hydrochloride solvate with 2-propanol

### Starting material:

Paroxetine base prepared as described in US6583287, title 91.6% (HPLC) - purity 99.5% (HPLC) containing about 9% of toluene, 273 g (corresponding to 250 g of pure paroxetine at 100%), anhydrous 2-propanol (H₂O K.F. 0.15%) 1250 ml, ascorbic acid 3.75 g, gaseous HCl in 2-propanol (title 20.4% w/w) 136 g (151.6 ml) corresponding to 1.00 mole/mole

### Procedure:

Paroxetine base was dissolved in 2-propanol in a flask and charged into a 1000 ml jacket reactor, equipped with mechanical stirring, thermometer and dropping funnel, under nitrogen. Ascorbic acid was then charged and, after 5 minutes from the addition, the solution changed from orange-red to light yellow. The HCl solution in 2-propanol was added dropwise at the temperature of 25 °C and, without seeding, there was abundant formation of crystals with turbidity of the solution. The addition of HCl was continued and the resulting suspension was maintained at 25°C under stirring overnight.

A sample of the admixture was filtered and dried in oven at 40°C overnight (from 28.7 g of product humid with 2-propanol, 19.8 g with a content of 2-propanol of 31 % w/w were obtained after drying).

The suspension was filtered on a buchner under nitrogen. The cake was washed with 2-propanol, pre-cooled at 5°C (2 x 100ml). The so obtained solvate was directly used in the next step.

### EXAMPLE 2

### Conversion of crystalline anhydrous paroxetine hydrochloride solvate with 2-propanol into paroxetine hydrochloride hemihydrate (static conditions)

After having removed, on the filter under vacuum, the excess of 2-propanol from the solvate prepared according to example 1, the solid cake was washed with water (2 x 100 ml).

The wet product was transferred from the buchner into a tray (450 g wet, 42% w/w of water) and left overnight at 40°C at room pressure, then under vacuum at 40°C for 5 hour and for 4 hours at 60°C.

260 g of paroxetine hydrochloride hemihydrate were thus obtained, with a molar yields of 98.3% (calculated considering the sample taken above for the analysis).

Analyses (in line with the reference standard of European Pharmacopeia): white powder, K.F. 2.46%, 2-propanol content: 761 ppm, toluene content: 16 ppm, total impurities (HPLC) < 0.05%, IR correlation vs EP std 0.9790, DSC peak 123.98°C, melting point (visual) 140.3°C (std EP 144°C), XRD in line with std EP, stability: a sample kept in the open air for 24 hours: K.F. 2.68%.

### EXAMPLE 3

### Conversion of crystalline paroxetine hydrochloride solvate with 2-propanol in paroxetine hydrochloride hemihydrate (washing with water-alcoholic admixtures)

Several conversion experiments, according to the procedure as described in the example 2, were performed. In these experiments 2-propanol containing increasing amount of water was used for washing the solid solvate, then the wet solid was left on the filter at about 40°C at room pressure for the specified times, with the following results:
3 a) content of water in the washing admixture = 3%, low conversion after 48 hours; DSC: two peaks; 2-propanol >5000ppm
3 b) content of water in the washing admixture = 5%, incomplete conversion at 48 hours; DSC: two peaks ; 2-propanol about 5000 ppm
3c) content of water in the washing admixture = 20%, complete conversion in 6 hours; DSC: one peak 123.98°C of the hemihydrate; 2-propanol = 2500-4800 ppm

### EXAMPLE 4: comparative experiments

### Preparation of paroxetine hydrochloride hemihydrate

Comparative preparations of paroxetine hydrochloride hemihydrate starting from the same paroxetine base, obtained as described in US6583287, were performed.

In these experiments the classical procedure of dissolution of the base, formation of the hydrochloride hemihydrate under non-anhydrous conditions and crystallization (Beecham process US4721723) was compared with the process of the present invention, substantially comprising the dissolution of the base, precipitation of the anhydrous hydrochloride solvate under anhydrous conditions followed by its conversion into the hemihydrate by hydration in the solid state.

The preparation of paroxetine hydrochloride hemihydrate, in line with the present invention, (process 4a) was performed according to the examples 1, 2 or 3 above, while the preparation according to the known art (Beecham, process 4b) was performed as described in the following:

### Process 4b (Beecham process) (example 4.4 table 1)

### Starting material:

Paroxetine base title 91.6% (HPLC) - purity 99.5% (HPLC) 109.2 g corresponding to 100 g of pure paroxetine (100%), 2-propanol (K.F. 0.15%) 720 ml, ascorbic acid 1.65 g, aqueous concentrated HCl (37%), 33.14 g (28 ml) corresponding to 1.10 moles/mole, neutral decolorizing charcoal g 2.7

### Procedure:

In a 1000 ml jacket reactor kept under nitrogen, equipped with mechanical stirrer, thermometer and dropping funnel at 25°C, 275 ml of 2-propanol and the concentrated HCl were charged. Paroxetine base was dissolved in the remaining 445 ml of 2-propanol and dropped into the reactor in 1 hour, with increase of the internal temperature up to 30°C. At the end of the addition a well stirrable suspension resulted, which was heated at reflux (int. temp. about 80°C) with complete dissolution of the solid. Ascorbic acid and decolorizing charcoal were then added, keeping the solution under stirring for 30 minutes; the solution was then cooled at 70-75 °C and filtered on dicalite.

The filter cake was washed with 50 ml of 2-propanol. The clear solution was transferred into a clean reactor and kept at 60°C, seeded with paroxetine hydrochloride hemihydrate (100 mg), kept under stirring for 30 minutes at 60°C and then cooled at 25° in about 2 hours. The suspension was kept at r.t. for a weekend, filtered at 20°C on a buchner and the resulting cake washed with 2 x 50ml of 2-propanol.

g 128 of wet product were obtained, product which was dried in oven under vacuum at 60°C for 20 hours, giving 107.0 g of paroxetine hydrochloride hemihydrate with a molar yields of 94.0%.

Analyses: white powder, K.F. 2.36%, 2-propanol 504 ppm, toluene 18 ppm, Total impurities (HPLC) 0.06%, IR correlation vs EP std 0.9842, DSC peak 128.73°C, melting point (visual) 141.3°C (std EP 144°C), XRD in line with std EP, stability: after 24 hours in the open air K.F. 2.87%.

The process according to the present invention provides for a product with higher yields (in particular see example 4.4, yields 98.4% of the present invention versus 94.0% of Beecham's process) and lower impurities content, as disclosed in the following table:

**Table 1**

| Example | | Total Impurities HPLC % | N-ethyl paroxetine | Max Impurity | Notes |
|---|---|---|---|---|---|
| 4.1 | Paroxetine base | 4.71 | N.d. | 2.35 | |
| | Process 4a | 0.53 | N.d. | 0.37 | |
| 4.2 | Paroxetine base | 1.21 | 0.53 | N.d. | |
| | Process 4a | 0.14 | 0.10 | N.d. | Washing with 2-propanol/water 20% |
| 4.3 | Paroxetine base | 1.45 | 0.90 | N.d. | |
| | Process 4a | 0.13 | 0.13 | N.d. | |
| | Process 4b | 0.25 | 0.15* | N.d. | * not complying with EP Pharmacopeia |
| 4.4 | Paroxetine base | 0.50 | <0.10 | 0.50** | ** Paroxetine, N-phenyl carbamate |
| | Process 4a | <0.05 | <0.02 | <0.05 | Molar yields: 98.4% |
| | Process 4b | 0.06 | 0.02 | 0.04 | Molar yields: 94.0% |
| 4.5 | Paroxetine base | 0.74 | 0.70 | 0.38 | |
| | Process 4a*** | 0.10 | 0.08 | <0.05 | *** pilot scale batch yields: 96% |

| | | | | | |
|---|---|---|---|---|---|
| N.d. = not determined | | | | | |

From the (non comparative) examples 4.1 and 4.2 it appears that the present process on the one hand, allows for a significant reduction of the impurities (ex. 4.1) and, on the other hand, can also be performed with water-alcoholic admixtures (ex. 4.2).

The example 4.3 shows that only the product prepared according to the present process complies with Pharmacopeia's requirements.

From the example 4.4 it appears that the present process is advantageous with respect to the Beecham's process even when the starting paroxetine base is highly pure (total impurities = 0.5%).

The (non comparative) example 4.5 demonstrates that the present process can be easily scaled up providing a product with good yields and a significantly low impurity content. This pilot batch was performed according to the following steps:
1) 31.5 Kg of paroxetine base were converted into paroxetine hydrochloride solvate with 2-propanol by addition of an anhydrous solution of gaseous HCl in 2-propanol to a solution of paroxetine base in anhydrous 2-propanol and toluene
2) the precipitated paroxetine hydrochloride solvate with 2-propanol was filtered in the filter-dryer, washed with 2-propanol, squeezed and partially dried
3) the resulting cake was washed with water in the filter-dryer (2 x 40 1) and heated under stirring at about 40°C, under nitrogen for about 5 hours
6) finally the product was dried under vacuum by gradually heating up to about 60°C up to K.F.= 2.2 - 2.7 % (theoretical value 2.4%)

According to the examples reported above the process for manufacturing paroxetine hydrochloride hemihydrate of the present invention appears to be easily applicable on industrial scale and particularly advantageous with respect to the methods known in the art both in terms of yields and purity.

## Claims

1. A process for manufacturing crystalline paroxetine hydrochloride hemihydrate which comprises the steps of:
a) wetting a crystalline anhydrous solvate of paroxetine hydrochloride with water or admixtures thereof with water-miscible solvents, and
b) heating the wet crystalline solvate of paroxetine hydrochloride, at a temperature between 30 and 60°C, up to its complete conversion into crystalline paroxetine hydrochloride hemihydrate.

2. The process according to claim 1 in which said crystalline anhydrous solvate of paroxetine hydrochloride comprises a solvent selected from the group consisting of methanol, ethanol, 1-propanol, 2-propanol, 1-butanol, acetone, acetonitrile, acetic acid and pyridine.

3. The process according to claim 2 in which said solvent is 2-propanol.

4. The process according to claim 3 in which said crystalline anhydrous solvate of paroxetine hydrochloride with 2-propanol is dried up to a 2-propanol content lower than 20% w/w.

5. The process according to claim 1 in which said wetting is performed with water.

6. The process according to claim 1 in which said wetting is performed with an admixture of water with a water-miscible solvent, being that solvent selected form the group consisting of methanol, ethanol, 1-propanol, 2-propanol, 1-butanol, acetone, acetonitrile, acetic acid and pyridine.

7. The process according to claim 6 in which said solvent is 2-propanol.

8. The process according to claim 6 in which said admixture of water with a water-miscible solvent comprises at least about 5% w/w of water.

9. The process according to claim 1 in which said crystalline anhydrous solvate of paroxetine hydrochloride is wetted by washing on a filter.

10. The process according to claim 1 in which said wetting is performed with water or admixtures thereof with water-miscible solvents cooled at a temperature lower than 20°C.

11. The process according to claim 1 in which said heating is performed at a temperature from 30°C to 60°C.

12. The process according to claim 11 in which said temperature is from 35 to 45°C.

13. The process according to claim 1 in which said heating is performed under an inert atmosphere.

14. The process according to claim 1 in which said wetting and said heating are performed on a filter dryer.

15. The process according to claim 1 in which said heating is performed under mixing conditions.

16. A process for manufacturing crystalline paroxetine hydrochloride hemihydrate which comprises the steps of:
a) wetting crystalline anhydrous solvate of paroxetine hydrochloride with 2-propanol with water on a filter dryer, and
b) heating the wet crystalline solvate of paroxetine hydrochloride, at a temperature between 35 and 45°C up to its complete conversion into crystalline paroxetine hydrochloride hemihydrate.

## Patentansprüche

1. Ein Verfahren zur Herstellung von kristallinem Paroxetin-Hydrochlorid-Halbhydrat bestehend aus folgenden Schritten: a) Benetzung von wasserfreiem, kristallinem Paroxetin-Hydrochlorid-Solvat mit Wasser oder einer Mischung aus Wasser und wassermischbaren Lösungsmitteln, und b) Erwärmen des nassen Paroxetin-Hydrochlorid-Solvats auf eine Temperatur zwischen 30 und 60 °C, bis zu seiner vollständigen Umwandlung in kristallines Paroxetin-Hydrochlorid-Halbhydrat.

2. Das Verfahren nach Patentanspruch 1, in dem das genannte wasserfreie, kristalline Paroxetin-Hydrochlorid-Solvat ein ausgewähltes Lösungsmittel aus der Gruppe von Methanol, Ethanol, 1-Propanol, 2-Propanol, 1-Butanol, Aceton, Acetonitril, Essigsäure und Pyridin enthält.

3. Das Verfahren nach Patentanspruch 2, in dem das genannte Lösungsmittel 2-Propanol ist.

4. Das Verfahren nach Patentanspruch 3, in dem das genannte wasserfreie, kristalline Paroxetin-Hydrochlorid-Solvat mit 2-Propanol auf einen 2-Propanol-Gehalt von weniger als 20 % Massenanteil getrocknet wird.

5. Das Verfahren nach Patentanspruch 1, in dem das genannte Benetzen mit Wasser durchgeführt wird.

6. Das Verfahren nach Patentanspruch 1, in dem das genannte Benetzen mit einer Mischung aus Wasser und einem wassermischbaren Lösungsmittel aus der Gruppe Methanol, Ethanol, 1-Propanol, 2-Propanol, 1-Butanol, Aceton, Acetonitril, Essigsäure und Pyridin durchgeführt wird.

7. Das Verfahren nach Patentanspruch 6, in dem das genannte Lösungsmittel 2-Propanol ist.

8. Das Verfahren nach Patentanspruch 6, in dem die genannte Mischung aus Wasser und einem wassermischbaren Lösungsmittel mindestens 5 % Massenanteil Wasser enthält.

9. Das Verfahren nach Patentanspruch 1, in dem das genannte wasserfreie, kristalline Paroxetin-Hydrochlorid-Solvat durch Waschen auf einem Filter benetzt wird.

10. Das Verfahren nach Patentanspruch 1, in dem das genannte Benetzen mit auf unter 20 °C abgekühltem Wasser bzw. einer auf unter 20 °C abgekühlten Mischung aus Wasser und wassermischbaren Lösungsmitteln erfolgt.

11. Das Verfahren nach Patentanspruch 1, in dem das genannte Erwärmen auf eine Temperatur zwischen 30 °C und 60 °C erfolgt.

12. Das Verfahren nach Patentanspruch 11, in dem die genannte Temperatur zwischen 30 °C und 45 °C liegt.

13. Das Verfahren nach Patentanspruch 1, in dem das genannte Erwärmen unter Inertgas-Atmosphäre durchgeführt wird.

14. Das Verfahren nach Patentanspruch 1, in dem das genannte Benetzen und das genannte Erwärmen auf einem Filtertrockner erfolgt.

15. Das Verfahren nach Patentanspruch 1, in dem das genannte Erwärmen unter gemischten Bedingungen erfolgt.

16. Ein Verfahren zur Herstellung von kristallinem Paroxetin-Hydrochlorid-Halbhydrat bestehend aus folgenden Schritten: a) Benetzung von wasserfreiem, kristallinem Paroxetin-Hydrochlorid-Solvat mit 2-Propanol und Wasser auf einem Filtertrockner; b) Erwärmen des nassen, kristallinen Paroxetin-Hydrochlorid-Solvats auf eine Temperatur zwischen 30 und 45 °C, bis zu seiner vollständigen Umwandlung in kristallines Paroxetin-Hydrochlorid-Halbhydrat.

## Revendications

1. Une procédure de fabrication de chlorhydrate de paroxétine hémihydraté cristallin qui comprend les étapes suivantes : a) humidification du solvate anhydre cristallin de chlorhydrate de paroxétine dans de l'eau ou mélange d'eau et de solvants miscibles dans l'eau, et b) chauffage du solvate cristallin humidifié de chlorhydrate de paroxétine, à une température comprise entre 30 et 60° C, jusqu'à son entière conversion en chlorhydrate de paroxétine hémihydraté cristallin.

2. La procédure conformément à la revendication 1 dans laquelle ce solvate anhydre cristallin de chlorhydrate de paroxétine comprend un solvant sélectionné d'un groupe composé de méthanol, éthanol, 1-propanol, 2-propanol, 1-butanol, acétone, acétonitrile, acide acétique et pyridine.

3. La procédure conformément à la revendication 2 dans laquelle ce solvant est 2-propanol.

4. La procédure conformément à la revendication 3 dans laquelle ce solvate anhydre cristallin de chlorhydrate de paroxétine avec 2-propanol est séché jusqu'à obtenir un contenu de 2-propanol inférieur à 20% poids humide.

5. La procédure conformément à la revendication 1 dans laquelle cette humidification est réalisée avec de l'eau.

6. La procédure conformément à la revendication 1 dans laquelle cette humidification est réalisée avec un mélange d'eau et de solvant miscible dans l'eau, ce solvant étant sélectionné d'un groupe composé de méthanol, éthanol, 1-propanol, 2-propanol, 1-butanol, acétone, acétonitrile, acide acétique et pyridine.

7. La procédure conformément à la revendication 6 dans laquelle ce solvant est 2-propanol.

8. La procédure conformément à la revendication 6 dans laquelle ce mélange d'eau et de solvant miscible dans l'eau comprend au moins environ 5% poids humide d'eau.

9. La procédure conformément à la revendication 1 dans laquelle ce solvate anhydre cristallin de chlorhydrate de paroxétine est humidifié par lavage à travers un filtre.

10. La procédure conformément à la revendication 1 dans laquelle cette humidification est réalisée avec de l'eau ou des mélanges d'eau et de solvants miscibles dans l'eau refroidis à une température inférieure à 20° C.

11. La procédure conformément à la revendication 1 dans laquelle ce chauffage est réalisé à une température comprise entre 30 et 60° C.

12. La procédure conformément à la revendication 11 dans laquelle cette température est comprise entre 35 et 45° C.

13. La procédure conformément à la revendication 1 dans laquelle ce chauffage est réalisé dans une atmosphère inerte.

14. La procédure conformément à la revendication 1 dans laquelle cette humidification et ce chauffage sont réalisés dans un séchoir à filtre.

15. La procédure conformément à la revendication 1 dans laquelle ce chauffage est réalisé dans des conditions de mélange.

16. Une procédure pour la fabrication de chlorhydrate de paroxétine hémihydraté cristallin qui comprend les étapes suivantes : a) humidification du solvate anhydre cristallin de chlorhydrate de paroxétine avec 2-propanol, avec de l'eau dans un séchoir à filtre et b) chauffage du solvate cristallin humidifié de chlorhydrate de paroxétine, à une température comprise entre 35 et 45° C jusqu'à son entière conversion en chlorhydrate de paroxétine hémihydraté cristallin.
